# EUROPÄISCHE PATENTSCHRIFT

(11) **EP 0 779 807 B1**
(45) Veröffentlichungstag und Bekanntmachung des Hinweises auf die Patenterteilung: **03.11.1999**
(21) Anmeldenummer: 95931985.6
(22) Anmeldetag: 04.09.1995
(51) Int. Cl.: A61K 9/44

(54) **VERFAHREN ZU hERSTELLUNG EINER MANTELTABLETTE MIT SPITZEM KERN**
PROCESS OF PREPARING A LAMINATED TABLET WITH POINTED CORE
PROCEDE DE PREPARATION D'UN COMPRIME ENROBE AVEC NOYAU POINTU

(30) Priorität: 06.09.1994 DE 4431653
(43) Veröffentlichungstag der Anmeldung: 25.06.1997
(73) Patentinhaber: LTS LOHMANN Therapie-Systeme GmbH, 56567 Neuwied (DE)
(72) Erfinder: CREMER, Karsten, D-53119 Bonn (DE)
(74) Vertreter: Flaccus, Rolf-Dieter, Dr.
(86) Internationale Anmeldenummer: EP9503474
(87) Internationale Veröffentlichungsnummer: WO9607401

(56) Entgegenhaltungen:
- EP-A- 0 127 282
- EP-A- 0 259 113
- EP-A- 0 259 219
- EP-A- 0 542 364
- WO-A-90/01925

## Beschreibung

Gegenstand der Erfindung ist ein Verfahren zur Herstellung einer Manteltablette zur kontrollierten Freisetzung von Wirkstoffen. Die Manteltablette soll es ermöglichen, in flüssigen Medien wie z. B. Körperflüssigkeiten Wirkstoffe verzögert und/oder mit gewünschtem Geschwindigkeitsprofil freizusetzen.

Insbesondere zum Zweck der oralen Verabreichung pharmazeutischer Wirkstoffe werden häufig Tabletten eingesetzt. Je nach therapeutischer Zielsetzung lassen sich dabei Tabletten mit schneller und solche mit gesteuerter Wirkstofffreisetzung unterscheiden. Eine gesteuerte Wirkstofffreisetzung wird z. B. dann angestrebt, wenn der Wirkstoff eine kurze biologische Halbwertzeit besitzt. In diesem Fall würde die Verabreichung in Form von schnell freisetzenden Tabletten zu erheblichen Schwankungen der Wirkstoff-Plasmakonzentrationen führen, es sei denn, es würden kleine Dosen in kurzen Abständen eingenommen. Erfahrungsgemäß halten Patienten vorgeschriebene Einnahmefrequenzen diese Art selten ein, was dann zum Therapieversagen führt. Die Einnahme von Tabletten, deren Wirkstofffreisetzung dahingehend gesteuert wird, daß sie beispielsweise über mehrere Stunden gleichmäßig verzögert eintritt, kann bei geringer Einnahmefrequenz und dadurch verbesserter Patienten-Compliance die Schwankung der Wirkstoff-Plasmakonzentrationen gering halten. Auf diese Weise ist die therapeutisch notwendige Wirkstoffzufuhr sichergestellt, wohingegen die Gefahr durch besonders hohe Plasmakonzentrationsspitzen vermieden ist.

Eine retardierte Wirkstofffreisetzung läßt sich auf verschiedenen Wegen erreichen. Zum einen gelingt sie durch physikalisch-chemische Maßnahmen, denen ein Wirkstoff unterworfen wird. Zu solchen Maßnahmen zählen z.B. der Einsatz von Wirkstoff-Adsorbaten, schwerlöslichen Wirkstoffsalzen und Komplexen.

Eine größere Kontrolle über das Ausmaß der Retardierung wird in der Regel jedoch durch galenische Maßnahmen erzielt. Viele der bekannten Retardtabletten lassen sich den Matrixsystemen einerseits oder den Membransystemen andererseits zuordnen. Matrixsysteme enthalten Wirkstoffe in gelöster und suspendierter Form, seltener auch in Form eines multipartikulären pharmazeutischen Zwischenproduktes. Die Freisetzung geschieht entweder durch Diffusion von Wirkstoffen aus der Matrix oder durch kontinuierliche, an den Randzonen beginnende Erosion der Matrix. Membransysteme hingegen enthalten ein wirkstoffhaltiges Reservoir, welches mit einem für den Wirkstoff zumindest semipermeablen Überzug umhüllt ist. Dabei geschieht die Freisetzung mittels Diffusion des Wirkstoffs durch die Membran.

Die Freisetzungsgeschwindigkeit hängt bei diesen Systemen von verschiedenen Einflußgrößen ab. Bei Matrixtabletten gehören dazu u. a. spezifische Eigenschaften der verwendeten Hilfssubstanzen wie Molmasse, Löslichkeit, Quellfähigkeit und Glasübergangstemperatur, aber auch Wirkstoffkonzentration und geometrische Form der Matrix. Bei der Freisetzung durch Diffusion aus einer Matrix zählen die Größe der aktiven Oberfläche, das Matrixvolumen, der Diffusionskoeffizient, die Konzentration und die Löslichkeit des Wirkstoffes in der Matrix, die Porosität und Tortuosität der Matrix, sowie der Diffusionswiderstand zwischen Matrix und einem flüssigen Umgebungsmedium zu dem wesentlichen Faktoren. Umhüllte Tabletten setzen Wirkstoffe mit einer Geschwindigkeit frei, die in erster Linie von der Größe der aktiven Oberfläche, der Permeabilität des Wirkstoffes durch die Membran und vom Konzentrationsgefälle zu beiden Seiten der Membran abhängt.

Mit herkömmlichen Matrix- und Filmtabletten läßt sich die Freisetzungsgeschwindigkeit nur begrenzt steuern. Die Verwirklichung einer gleichmäßig retardierten Freisetzung stößt in beiden Fällen auf Schwierigkeiten. Bei erodierbaren Matrices verändert sich - je nach Form der Matrix - die Freisetzungsgeschwindigkeit im Verlauf der Freisetzung mehr oder weniger stark aufgrund der Veränderung der erodierbaren Oberfläche. Bei Diffusionsmatrices bildet sich dagegen im Freisetzungsverlauf eine durch zunehmende Wirkstoffdepletion wachsende Diffusionsschicht mit der Folge aus, daß sich die Freisetzungsgeschwindigkeit in Abhängigkeit von t^{½} verringert (Higuchi, J. Pharm. Sci. 50, S. 874, 1961).

Eine verbesserte Kontrolle des Freisetzungsprofiles wurde durch die Einführung weiterer Steuermechanismen erlangt. Beispielsweise beschreibt EP-A 0 432 607 eine Mehrschichttablette, deren wirkstoffhaltige Matrix eine der Schichten darstellt, welche durch Hilfsstoffschichten teilweise bedeckt ist.
Die Verwendung geometrischer Elemente ist in US 3 924 622 beschrieben, wobei es sich bei der hierin beanspruchten Vorrichtung zur gesteuerten Wirkstoffabgabe nicht um eine Tablette handelt. Jedoch wird hier das geometrische Steuerprinzip der kompensatorischen Flächenvergrößerung beschrieben, mit welchem den verlangsamenden Faktoren der Freisetzung - Verlängerung des Diffusionsweges, Wirkstoffdepletion etc. - entgegengewirkt wird. Die Vorrichtung ist so gestaltet, daß sie ein Wirkstoffreservoir mir einer definierten, gleichbleibenden Öffnung besitzt, durch die der Wirkstoff nach außen tritt. Die Freisetzung geschieht durch Erosion des Reservoirs. Die Form des Reservoirs ist so gewählt, daß sich die Erosionsfläche mit der im Laufe der Freisetzung zunehmenden Entfernung der Erosionsfront von der Öffnung kontinuierlich vergrößert.

Einem ähnlichen Steuermechanismus unterliegt die in EP-A 0 542 364 beschriebenen Vorrichtungen. Auch bei diesen vergrößert sich im Laufe der Freisetzung eine Fläche, nämlich die Diffusionsfront. Dies hat denselben aufrechterhaltenden Effekt auf die Freisetzungsgeschwindigkeit wie das geometrische Element in US 3 924 622. Ausdrücklich wird auf die Ausgestaltungsmöglichkeit als Tablette hingewiesen, wobei die Anforderungen an die Vorzugsform eher komplex sind.

EPA 0 259 219 beschreibt eine umhüllte Tablette mit einer zentralen Öffnung, durch welche der Wirkstoff aus dem Tablettenkern nach außen freigesetzt wird. Die Dicke des Tablettenkernes nimmt von der zentralen Öffnung zur Peripherie hin zu, wodurch analog zu den oben beschriebenen Systemen die sich im Freisetzungsverlauf vergrößernde Distanz zwischen Erosions- oder Diffusionsfront zur Öffnung durch eine Flächenvergrößerung kompensiert wird.

Eine Vorrichtung zur kontrollierten Freisetzung von Wirkstoff ist auch in der WO 90/01925 beschrieben. Diese weist einen erodierbaren und mindestens einen Wirkstoff enthaltenden Kern zur kontrollierten Wirkstofffreisetzung auf. Dieser ist in einer weitgehend erosionsresistenten Mantelschicht mit einer Öffnung angeordnet, an die der Kern heranreicht.
Die Vorrichtung wird durch Umhüllung eines Kerns, der z.B. in Form einer konvexen oder bikonvexen Tablette vorliegt, durch Sprüh-, Tauch- oder Tablettierverfahren hergestellt. Die Öffnung wird nachträglich mit einem geeigneten Werkzeug in die Umhüllung eingebracht. Im Falle der Manteltablettierung kann die Öffnung durch asymetrisches bzw. exzentrisches Positionieren beim Aufpressen der Mantelschicht erzeugt werden.

Ein Kompensationsmechanismus anderer Art wird in der unveröffentlichten deutschen Patentanmeldung P 43 41 442.7 beschrieben. Hierbei handelt es sich um eine Vorrichtung mit einer wirkstoffhaltigen Matrix, welche zunächst teilweise durch erodierbare Hilfsstoffschichten mit Dickegradienten bedeckt ist, wobei diese Hilfsstoffschicht im Zuge der Freisetzung erodieren und dadurch eine Vergrößerung der freisetzungsaktiven Matrixoberfläche bewerkstelligen.

Eine ähnlich wirksame Steuerung der Freisetzungsgeschwindigkeit läßt sich mit osmotischen Systemen erreichen. Diese enthalten ein umhülltes Wirk- und Hilfsstoffreservoir, in welchem sich nach Zutritt von Wasser ein osmotischer Druck aufbaut. Die Membranen, welche die Wirkstoffreservoirs umgeben, sind semipermeabel; sie ermöglichen den Eintritt von Wasser, sind für Wirkstoffe jedoch undurchlässig, besitzen aber eine fast mikroskopisch kleine Öffnung, durch welche eindiffundiertes Wasser zusammen mit gelöstem Wirkstoff austreten kann. Mit solchen osmotischen Systemen konnten mitunter über längere Zeit konstante Freisetzungsgeschwindigkeiten erzielt werden (Theuwes, Pharm. Int. 5, 293, 1984).

Sämtliche dieser Vorrichtungen zur kontrollierten Wirkstofffreisetzung besitzen gegenüber herkömmlichen, einfach aufgebauten Retardtabletten den Vorteil, die Freisetzungsgeschwindigkeit in wesentlich höherem Ausmaß steuern zu können. Dies gilt für die präzise arbeitenden osmotischen Systeme insbesondere dann, wenn eine Freisetzung nullter Ordnung angestrebt wird. Die Vorrichtungen mit geometrischen Steuerelementen sind in dieser Hinsicht variabler und lassen sich eher auch zum Erzielen anderer Freigabeprofile anwenden.

Jedoch besitzen viele der vorgeschlagenen Ausführungsformen gegenüber den konventionellen Retardtabletten den Nachteil, daß sie sehr aufwendig in der Fertigstellung sind. Dies gilt umso mehr für die osmotischen Systeme, bei denen die konventionelle Tablettentechnologie bisher nicht eingesetzt werden kann und das Einbringen einer Öffnung in der Umhüllung größte Präzision und Reproduzierbarkeit erfordert und nur mit Hilfe teurer Technologien und beschränkter Fertigungseffizienz bewerkstelligt werden kann.

Der Erfindung liegt die Aufgabe zugrunde, ein Verfahren zur Herstellung von Tabletten zur kontrollierten Freisetzung von Wirkstoffen zu schaffen, welche geometrische Elemente zur Steuerung der Freisetzungsgeschwindigkeit besitzt, nämlich eine Öffnung, durch welche Wirkstoff austritt und dabei mit zunehmendem Abstand zu dieser Öffnung eine sich vergrößernde Diffusions- bzw. Erosionsfront ausgebildet, das hocheffizient ist und mit dem die Tabletten einfach und in großen Stückzahlen zu fertigen sind.

Erfindungsgemäß wird zunächst der wirkstoffhaltige Tablettenkern mit einem schmal oder spitz zulaufenden Endbereich geformt und dabei aus Pulver oder Granulat gepreßt. Der gepreßte Tablettenkern wird anschließend einer zur exakten Kernpositionierung geeigneten Manteltablettenpresse zugeführt und dort derart in eine bereits teilbefüllte Matrizenöffnung gedrückt, daß sein schmal oder spitz zulaufendes Ende an den Rand der vorgesehenen Matrizenöffnung heranreicht. Danach wird er mit wieterem Pulver oder Granulat zusammengepreßt, wobei mit dem Preßvorgang in der Mantelschicht eine Öffnung ausgebildet wird, an die der Tablettenkern mit seinem spitz zulaufenden Ende heranreicht. Durch die Öffnung wird bei Anwendung der Tablette der Wirkstoff freigesetzt.

Die Erfindung schafft mit diesem Verfahren erstmals die Möglichkeit, Manteltabletten mit einer definierten, gegenüber den Ausmaßen des Kernes vergleichsweise kleinen Öffnung zu versehen und zur Steuerung der Freisetzungsgeschwindigkeit einzusetzen, und diese mit einer üblichen, hocheffizienten Tablettentechnologie einfach, präzise und mit ökonomischen Mitteln in großen Stückzahlen herzustellen.

Durch die Form des Kerns, die in Abhängigkeit von den Anforderungen an das Freisetzungsprofil im Einzelfall festgelegt wird, läßt sich bestimmen, mit welchem Gradienten die freisetzungsrelevante Querschnittsfläche - also in diesem Falle die Erosions- oder Diffusionsfront - mit der Distanz zu Öffnung zunimmt.

Mit Vorteil kann sich die Querschnittsfläche des Kerns mit zunehmender Entfernung von der Öffnung des Mantels nach einer Funktion erster oder zweiter Ordnung stetig ändern. Es kann aber auch von der Maßnahme Gebrauch gemacht sein, daß sich die Querschnittsfläche des Kerns mit zunehmender Entfernung von der Öffnung sprunghaft ändert.
Eine weitere Ausgestaltung sieht vor, daß der Kern wenigstens zwei unterschiedliche Wirkstoffe, z.B. für Voroder Nachbehandlung eines Krankheitszustandes, enthält und diese entweder in homogener Mischung oder in unterschiedlichen Schichten der Kerns vorliegen. Auch können in der Manteltablette mehrere Kerne mit unterschiedlichen Wirkstoffen untergebracht werden.

Die Form der Manteltablette kann der Form des Tablettenkerns angeglichen werden und dieser kann aus mindestens 70 % der Masse der Manteltablettegebildet sein, wodurch der Anteil des Wirkstoffs an der Gesamtmasse entsprechend hoch gewählt werden kann.
Schließlich kann auch der Mantelschicht Wirkstoff zugegeben werden.

Im folgenden soll die Erfindung näher erläutert werden.

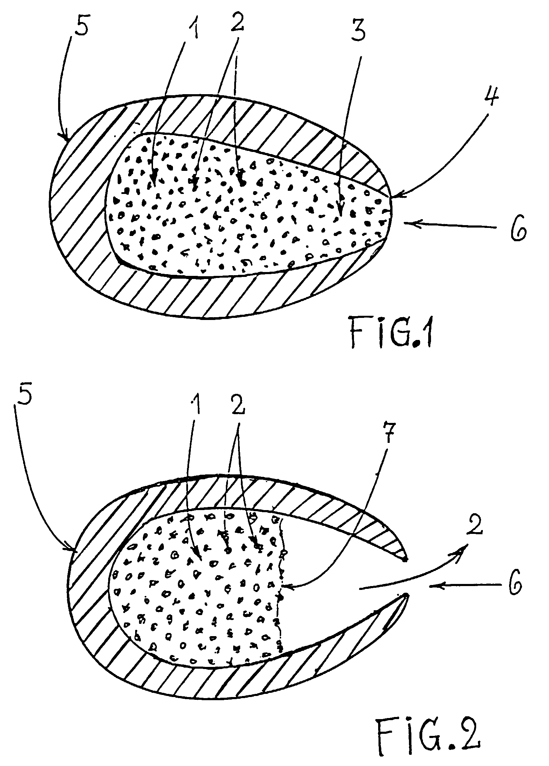

Figur 1 zeigt den Aufbau einer erfindungsgemäßen Tablette. Der Tablettenkern (1), der Wirkstoff (2) enthält, ist zum Endbereich (3) spitz zulaufend ausgebildet und reicht mit seinem spitz zulaufenden Ende an den Außenrand (4) der Tablette heran. Da an dieser Stelle die den Kern (1) umgebende Masse des Mantels (5) der Tablette unterbrochen ist, ergibt sich eine Öffnung (6), durch welche Wirkstoff (2) aus der Tablette freigesetzt werden kann.

Im Verlaufe der Freisetzung, die entweder durch Diffusion aus dem Tablettenkern (1) oder durch Erosion des Tablettenkerns (1) geschieht, wird zunächst Wirkstoff (2) aus dem am Tablettenaußenrand (4) liegenden Endbereich (3) des Kerns (1) freigegeben. Infolgedessen wird eine sich kontinuierlich von der Öffnung (6) entfernende Diffusions- oder Erosionsfront (7) ausgebildet, deren Größe, wie in Figur 2 gezeigt, mit der Querschnittsfläche des Kernes an ihrer Position etwa identisch ist. Mit zunehmender Entfernung der Erosionsfront (7) von der über den Freigabeverlauf annähernd konstant bleibenden Öffnung (6) nimmt das Ausmaß der erodierbaren Fläche beispielsweise nach einer Funktion erster oder zweiter Ordnung oder auch sprunghaft zu.

Die Steuerung der Freisetzungsgeschwindigkeit ist umso wirksamer, je formstabiler der Mantel (5) zumindest über einen wesentlichen Teil der Freisetzungsdauer und je weniger permeabel er für Wirkstoff ist. Durch diese Bedingungen wird sichergestellt, daß die Wirkstofffreisetzung überwiegend an der Stelle geschieht, an welcher sich die Öffnung befindet. Die Erfüllung dieser Bedingungen hängt von der Wahl der Hilfsstoffe und von den Herstellungsparametern ab.

Grundsätzlich kann der Mantel (5) aus physiologisch unbedenklichen Polymeren, Wachsen, wachsartigen Substanzen, Fetten, Fettsäuren, Fettalkoholen oder anderen pharmazeutisch einsetzbaren Tablettierhilfsstoffen, ggf. im Gemisch mit weiteren Hilfsstoffen wie z.B. Antioxidantien, Farbstoffen, Pigmenten, Aromen, Fließ-, Trenn- und Schmiermitteln, Netzmitteln, Löslichkeitsverbesserern, Hydrophilisierungsmitteln, Füllstoffen, Substanzen zur Einstellung des pH-Wertes, u.s.w. aufgebaut sein. Unabhängig von der Löslichkeit des Materials, aus dem der Mantel (5) gebildet wird, muß die Lösungsgeschwindigkeit gering sein, um einen Formerhalt gewährleisten zu können. Die Lösungsgeschwindigkeit wiederum hängt nicht nur von der Löslichkeit des Materials ab, sondern auch von der Kraft, mit der das Material komprimiert wird. Auch gut lösliche Materialien lassen sich zu einem sehr langsam zerfallenden Mantel pressen, wenn eine entsprechend hohe Preßkraft eingestellt wird. Ungünstig für den Formerhalt des Mantels (5) wirken sich hingegen in der Tablettentechnologie als Zerfallsbeschleuniger eingesetzte Substanzen aus. Bei diesen handelt es sich in der Regel um quervernetzte, hydrophile Polymere mit stark quellenden Eigenschaften. Beispiele hierfür sind Crospovidone und Croscarmellose.

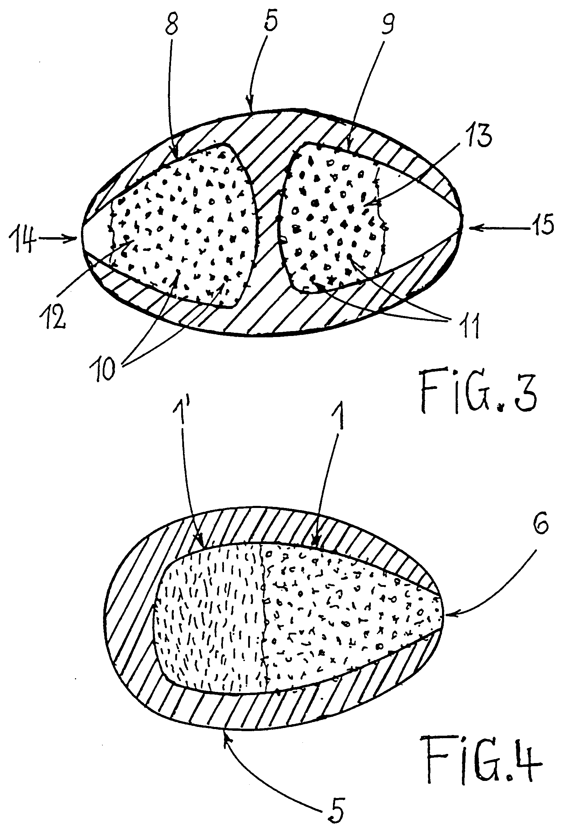

Die Erfindung erlaubt auch das Inkorporieren von mehr als einem Wirkstoff. So ist es beispielsweise möglich, wie Figur 3 zeigt, zwei Tablettenkerne (8) und (9) mit unterschiedlichen Wirkstoffen (10) und (11) pro Tablette einzulegen, deren schmal oder spitz zulaufende Endbereiche (12) und (13) an unterschiedlichen Stellen (14) und (15) an die Außenwand (4) der Tablette heranreichen. Ebenso kann eine Tablette nach Abbildung 1 erfindungsgemäß einen wirkstoffhaltigen Kern (1) mit mehr als einem Wirkstoff (2) enthalten. Schließlich kann die Tablette in einer Zone wie z.B. im Mantel (5) oder in einer zusätzlich eingebrachten separaten Komponente ebenfalls Wirkstoff enthalten, welcher dann allerdings nicht dem oben beschriebenen Mechanismus der Freisetzungskontrolle unterliegt.

Für die Einnahme besonders vorteilhaft ist die Beschränkung der Tablettengröße auf die Mindestabmessungen. In der Regel wird man zumindest bemüht sein, die Größe des Mantels (5) dadurch zu beschränken, daß man ihn relativ zum Tablettendurchmesser dünn gestaltet, was durch eine Angleichung der Kernform an die Tablettenform unterstützt werden kann. Anzustreben ist dabei eine Begrenzung der Mantelmasse auf höchstens 30% der Gesamtmasse der Tablette.

Eine Tablette mit einem zwei Teile (1) bzw. (1') aufweisenden Kern zeigt die Figur 4. Die Kernteile (1, 1') enthalten unterschiedliche Wirkstoffe (2) bzw. (2'). Es kann sich dabei um Wirkstoff zur Vor- und anschließenden Nachbehandlung eines Krankheitszustandes, z.B. einer Irritation der Magenschleimhaut o.ä. handeln.

Wenn auch der Hauptanwendungsbereich der erfindungsgemäßen Tablette im pharmazeutischen Einsatz liegt, so ist auch ihre Verwendung zur kontrollierten Freisetzung von Düngstoffen und Pflanzenschutzmitteln sehr vorteilhaft, da sie durch eine verlängerte Wirkdauer die Arbeit des wiederholten Aufbringens konventionell zu dosierender Düngstoffe und Pflanzenschutzmittel erspart. Ebenso vorteilhaft ist der Einsatz der Tablette zur Freisetzung von mikrobiellen Stoffen, insbesondere in Spül- und Waschprozessen.

## Patentansprüche

1. Verfahren zur Herstellung einer Manteltablette zur kontrollierten Freisetzung von Wirkstoff, mit einem erodierbaren und mindestens einen Wirkstoff (2) enthaltenden Tablettenkern (1) und einer weitgehend erosionsresistenten Umhüllung aus einer Mantelschicht (5) mit einer Öffnung,
**dadurch gekennzeichnet,**
daß zuerst der wirkstoffhaltige Tablettenkern (1) mit einem schmal oder spitz zulaufenden Endbereich (3) geformt und dabei aus Pulver oder Granulat gepreßt und sodann dieser einer Manteltablettenpresse zugeführt wird, welche ihn derart in eine bereits teilbefüllte Matrizenöffnung drückt, daß sein schmal oder spitz zulaufendes Ende (3) an den Rand der vorgesehenen Matrizenöffnung heranreicht, und daß er daraufhin mit weiterem Pulver oder Granulat zusammengepreßt wird, wobei in der Mantelschicht (5) eine Öffnung zugleich mit den Preßvorgang ausgebildet wird, an die der Kern (1) mit seinem spitz zulaufenden Ende (3) heranreicht.

2. Verfahren nach Anspruch 1, dadurch gekennzeichnet, daß die Querschnittsfläche des Kerns (1) mit zunehmender Entfernung von der Öffnung (6) der Mantelschicht (5) nach einer Funktion erster oder zweiter Ordnung stetig geändert wird.

3. Verfahren nach Anspruch 1 oder 2, dadurch gekennzeichnet, daß die Querschnittsfläche des Kerns (1) mit zunehmender Entfernung von der Öffnung (6) sprunghaft geändert wird.

4. Verfahren nach einem oder mehreren der Ansprüche 1 bis 3, dadurch gekennzeichnet, daß die Form der Manteltablette der Form des Tablettenkerns (1) angeglichen wird und der Kern (1) aus mindestens 70% der Masse der Manteltablette gebildet wird.

5. Verfahren nach einem oder mehreren der Ansprüche 1 bis 4, dadurch gekennzeichnet, daß der Kern (1) aus wenigstens zwei unterschiedlichen Wirkstoffen gebildet wird und diese entweder in homogener Mischung oder in unterschiedlichen Schichten zugegeben werden.

6. Verfahren nach einem oder mehreren der Ansprüche 1 bis 5, dadurch gekennzeichnet, daß auch der Mantelschicht (5) Wirkstoff (2) zugegeben wird.

7. Verfahren nach einem oder mehreren der Ansprüche 1 bis 6, dadurch gekennzeichnet, daß der Manteltablette wenigstens zwei Kerne (8,9) mit unterschiedlichen Wirkstoffen zugegeben werden.

8. Verwendung der Manteltablette nach den vorhergehenden Ansprüchen 1 bis 7 zur Freisetzung von Pflanzenschutzmitteln.

9. Verwendung der Manteltablette nach den Ansprüchen 1 bis 7 zur Freisetzung von Düngestoffen.

10. Verwendung der Manteltablette nach den Ansprüchen 1 bis 7 zur Freisetzung von antimikrobiellen Stoffen in Spül- oder Waschprozessen.

11. Verwendung der Manteltablette nach den Ansprüchen 1 bis 7 zur Herstellung eines Arzneimittels.

## Claims

1. Method for producing a dry-coated tablet for controlled release of active substance, having an erodible core tablet (1) containing at least one active substance (2), and a substantially erosion-resistant shell consisting of a dry-coated layer (5) with an opening, characterized in that the core tablet (1) containing active substance is first shaped with a narrowing or tapering end region (3) and in so doing is pressed from powder or granules, and this is then fed to a dry-coated tablet Press, which presses it into an already partially filled die opening so that its narrowing or tapering end (3) extends as far as the edge of the die opening provided, and in that it is thereafter compacted with further powder or granules, an opening being made in the dry-coated layer (5) simultaneously with the pressing operation, to which opening the core tablet (1) extends via its tapering end (3).

2. Method according to Claim 1, characterized in that the cross-sectional area of the core tablet (1) is changed continuously, on the basis of a function of first or second order, as its distance from the opening (6) increases.

3. Method according to Claim 1 or 2, characterized in that the cross-sectional area of the core tablet (1) is changed discontinuously as its distance from the opening (6) increases.

4. Method according to one or more of Claims 1 to 3, characterized in that the shape of the dry-coated tablet is matched to the shape of the tablet core (1), and the tablet core (1) constitutes at least 70% of the weight of the dry-coated tablet.

5. Method according to one or more of Claims 1 to 4, characterized in that the core tablet (1) is formed by at least two different active substances and these are added either in homogeneous mixture or in different layers.

6. Method according to one or more of Claims 1 to 5, characterized in that active substance (2) is added to the dry-coated layer (5) too.

7. Method according to one or more of Claims 1 to 6, characterized in that at least two core tablets (8, 9) with different active substances are added to the dry-coated tablet.

8. Use of the dry-coated tablet according to preceding claims 1 to 7 for the release of plant protection agents.

9. Use of the dry-coated tablet according to Claims 1 to 7 for the release of fertilizers.

10. Use of the dry-coated tablet according to Claims 1 to 7 for the release of anti-microbial substances, in dishwashers or washing processes.

11. Use of the dry-coated tablet according to Claims 1 to 7 for producing a medicament.

## Revendications

1. Procédé pour la préparation d'un comprimé enrobé à des fins de libération contrôlée d'un principe actif comprenant un noyau de comprimé (1) apte à être érodé et contenant au moins un principe actif (2), et un enrobage résistant dans une large mesure à l'érosion, constitué d'une couche d'enrobage (5) munie d'une ouverture, caractérisé en ce qu'on forme d'abord le noyau de comprimé (1) contenant le principe actif avec une zone terminale (3) effilée ou pointue et en ce qu'on le comprime en l'occurrence à partir d'une poudre ou d'un produit de granulation, puis on l'achemine à une machine de fabrication de comprimé enrobé qui l'enfonce par pression dans une ouverture de matrice déjà partiellement remplie de telle sorte que son extrémité effilée ou pointue (3) s'étend jusqu'au bord de l'ouverture de matrice prévue, et en ce qu'on le comprime par la suite avec une quantité supplémentaire de poudre ou de produit de granulation, une ouverture étant pratiquée dans la couche d'enrobage (5) de manière simultanée avec le processus de compression, contre laquelle aboutit le noyau (1) avec son extrémité pointue (3).

2. Procédé selon la revendication 1, caractérisé en ce que l'aire de surface de la section du noyau (1), au fur et à mesure que l'on s'éloigne de l'ouverture (6) de la couche d'enrobage (5), est modifiée en continu conformément à une fonction du premier ou du deuxième ordre.

3. Procédé selon la revendication 1 ou 2, caractérisé en ce que l'aire de surface de la section du noyau (1), au fur et à mesure que l'on s'éloigne de l'ouverture (6), est modifiée en discontinu.

4. Procédé selon une ou plusieurs des revendications 1 à 3, caractérisé en ce qu'on adapte la forme du comprimé enrobé à la forme du noyau de comprimé (1), le noyau (1) représentant au moins 70% de la masse du comprimé enrobé.

5. Procédé selon une ou plusieurs des revendications 1 à 4, caractérisé en ce que le noyau (1) est formé par au moins deux principes actifs différents, ces derniers étant ajoutés, soit sous forme d'un mélange homogène, soit sous forme de couches différentes.

6. Procédé selon une ou plusieurs des revendications 1 à 5, caractérisé en ce qu'on ajoute un principe actif (2) également à la couche d'enrobage (5).

7. Procédé selon une ou plusieurs des revendications 1 à 6, caractérisé en ce qu'on ajoute au comprimé enrobé, au moins deux noyaux (8, 9) comprenant des principes actifs différents.

8. Utilisation du comprimé enrobé selon les revendications précédentes 1 à 7 pour la libération d'agents de protection des plantes.

9. Utilisation du comprimé enrobé selon les revendications 1 à 7 pour la libération d'engrais.

10. Utilisation du comprimé enrobé selon les revendications 1 à 7 pour la libération de substances antimicrobiennes dans des processus de rinçage ou de lavage.

11. Utilisation du comprimé enrobé selon les revendications 1 à 7 pour la préparation d'un médicament.
